# EUROPEAN PATENT APPLICATION

(11) **EP 4 534 655 A1**
(43) Date of publication of application: **09.04.2025**
(21) Application number: 23812060.4
(22) Date of filing: 17.05.2023
(51) Int. Cl.: C12N 5/09, C12N 5/071, A01N 1/02, G01N 33/50

(54) **METHOD FOR MANUFACTURING CUSTOMIZED TUMOR ORGANOID PANEL, AND ANTI-CANCER DRUG SCREENING SYSTEM USING PANEL MANUFACTURED BY METHOD**

(30) Priority: 24.05.2022 KR 20220063528; 06.03.2023 KR 20230029394
(71) Applicant: SG Medical, Inc., Seoul 05548 (KR)
(72) Inventor: CHO, Gun Sik, Seoul 07220 (KR); CHOI, Yun Sik, Anyang-si, Gyeonggi-do 14062 (KR); BAEK, Keum Jin, Suwon-si, Gyeonggi-do 16504 (KR); MUN, Hye Min, Seoul 05399 (KR); SEO, Yu Ri, Seoul 02200 (KR)
(74) Representative: Cabinet Becker et Associés
(86) International application number: PCT/KR2023/006668
(87) International publication number: WO 2023/229286

(57) **Abstract**

The present invention relates to a method for making various organoid combinations by selecting frozen tumor organoid columns on the basis of genetic information, and analyzing drugs by using same. Particularly, the present invention relates to: a method for manufacturing a customized tumor organoid panel in which tumor organoids are frozen, thawed and stabilized in a 96-well strip plate in sizes suitable for drug evaluation, and then various tumor organoids are combined such that drugs can be rapidly mass-screened; and an anti-cancer drug screening system using the panel manufactured by the method. If drugs are evaluated using the customized tumor organoid panel, according to the present invention, the difficulty of simultaneous evaluation, caused by a difference in tumor organoid growth rates, can be solved, and evaluation can be carried out using various tumor organoids, and thus mass screening is possible. In addition, tumor organoids of a specific diameter are frozen so that the survival rate of tumor organoids can be increased and drugs can be more rapidly evaluated, and thus the present invention can be effectively used as an anti-cancer drug screening method.

## Description

### [Technical Field]

The present invention relates to a method for making various tumor organoid combinations by selecting frozen tumor organoid columns on the basis of genetic information, and analyzing drugs by using same. Particularly, the present invention relates to: a method for manufacturing a customized tumor organoid panel in which tumor organoids are frozen, thawed and stabilized in a 96-well strip plate in sizes suitable for drug evaluation, and then various tumor organoids are combined such that drugs may be rapidly mass-screened; and an anti-cancer drug screening system using the panel manufactured by the method.

### [Background Art]

Cancer has a high death rate worldwide, and is the most common death cause next to cardiovascular disease in the Western society. In particular, due to the westernization of eating habits, the widespread consumption of high-fat foods, a rapid increase in environmental pollutants, and an increase in alcohol consumption, colon cancer, breast cancer, liver cancer, and prostate cancer tend to continuously increase, and due to the aging population, increased smoking population, and air pollution, lung cancer is increased. In this situation, the creation of anticancer substances has been urgently required by enabling the early prevention and treatment of cancer to contribute to enhance human health, improve the quality of health life, and promote human health care.

An organoid refers to a three-dimensional cell aggregate formed through self-renewal and self-organization from stem cells. The organoid is considered as an organ analogue that has a structure including cells similar to an organ in vitro and may reproduce some functions of the organ. The organoid may better embody the complexity of the tissue and cell-to-cell interactions than a conventional two-dimensional cell culture method and has been used in disease modeling, drug toxicity/efficacy evaluation, and drug exploration research due to cheaper cost than an animal experiment that raises ethical issues.

Since tumor organoids formed from a cancer tissue of a patient have different growth rates, it is difficult to culture all tumor organoids to be used according to the growth rates when attempting to conduct drug evaluations using various tumor organoids at the same time. Therefore, there is a difficulty in separately evaluating drugs according to the growth rates after culturing each tumor organoid.

Currently, Korean Publication No. 2020-0055673 discloses an organoid manufactured using a cell culture carrier and a method for evaluating drug toxicity using the same, and Korean Publication No. 2022-0018946 discloses a method for manufacturing a pancreatic cancer organoid. There is not disclosed a method for manufacturing a customized tumor organoid panel capable of screening anticancer drugs by simultaneously evaluating various organoids and enabling mass screening.

### [Disclosure]

### [Technical Problem]

An object of the present invention is to provide a method for manufacturing a customized tumor organoid panel including (a) culturing tumor organoids derived from a cancer patient; (b) manufacturing the cultured tumor organoids into a panel; (c) freezing the manufactured tumor organoid panel; and (d) combining the frozen tumor organoid panels after thawing.

Another object of the present invention is to provide a customized tumor organoid panel manufactured by the method.

Yet another object of the present invention is to provide an anti-cancer drug screening method using the manufactured customized tumor organoid panel.

### [Technical Solution]

In order to achieve the object, an aspect of the present invention provides a method for manufacturing a customized tumor organoid panel including (a) culturing tumor organoids derived from a cancer patient; (b) manufacturing the cultured tumor organoids into a panel; (c) freezing the manufactured tumor organoid panel; and (d) combining the frozen tumor organoid panels after thawing.

Another aspect of the present invention provides a customized tumor organoid panel manufactured by the method.

Yet another aspect of the present invention provides an anti-cancer drug screening method using the manufactured customized tumor organoid panel.

### [Advantageous Effects]

According to the present invention, if drugs are evaluated using the customized tumor organoid panel, the difficulty of simultaneous evaluation, caused by a difference in tumor organoid growth rates, can be solved, and evaluation can be carried out using various tumor organoids, and thus mass screening is possible. In addition, tumor organoids of a specific diameter are frozen so that the survival rate of tumor organoids can be increased and drugs can be more rapidly evaluated, and thus the present invention can be effectively used as an anti-cancer drug screening method.

### [Description of Drawings]

FIG. 1 is a diagram illustrating a method for manufacturing a customized tumor organoid panel and a process of performing anti-cancer drug screening after thawing.
FIG. 2 is a diagram illustrating results of observing under a microscope whether the shapes of tumor organoids change according to a diameter of the organoid after freezing a tumor organoid panel according to an example of the present invention and then thawing the panel after three months.
FIG. 3 is a graph showing results of measuring a difference in survival rate according to a diameter of tumor organoid using Calcein-AM and EthD-1 after freezing a tumor organoid panel according to an example of the present invention and then thawing the panel after three months.
FIG. 4 is a diagram illustrating a graph showing results of comparing culture periods before drug treatment in a conventional cryopreservation method and a customized tumor organoid panel freezing method.
FIG. 5 is a diagram illustrating a graph showing results of performing anticancer efficacy evaluation in lung cancer organoids according to a conventional cryopreservation method and a customized tumor organoid panel freezing method.
FIG. 6 is a diagram illustrating a graph showing results of performing anticancer efficacy evaluation in colon cancer organoids according to a conventional cryopreservation method and a customized tumor organoid panel freezing method.
FIG. 7 is a diagram illustrating a graph showing results of performing anticancer efficacy evaluation in liver cancer organoids according to a conventional cryopreservation method and a customized tumor organoid panel freezing method.
FIG. 8 is a diagram illustrating results of analyzing a correlation based on the results of performing anticancer efficacy evaluation in lung cancer organoids according to a conventional cryopreservation method and a customized tumor organoid panel freezing method.
FIG. 9 is a diagram illustrating results of analyzing a correlation based on the results of performing anticancer efficacy evaluation in colon cancer organoids according to a conventional cryopreservation method and a customized tumor organoid panel freezing method.
FIG. 10 is a diagram illustrating results of analyzing a correlation based on the results of performing anticancer efficacy evaluation in liver cancer organoids according to a conventional cryopreservation method and a customized tumor organoid panel freezing method.

### [Best Mode]

The present invention provides a method for manufacturing a customized tumor organoid panel including (a) culturing tumor organoids derived from a cancer patient; (b) manufacturing the cultured tumor organoids into a panel; (c) freezing the manufactured tumor organoid panel; and (d) combining the frozen tumor organoid panels after thawing.

In the present invention, the "organoid" means a three-dimensional cell structure prepared by three-dimensionally culturing or recombining cells, and is also called a "mini-organ" or "pseudo-organ". The organoids specifically include one or more cell types among various types of cells that constitute an organ or tissue, and needs to be able to reproduce the shape and function of the tissue or organ.

The organoids may be used for new drug development, disease treatment, and artificial organ development. When developing new drugs, conventional drug tests had limitations in that the results of animal experiments and clinical experiments were different from each other, such as side effects that had not been found in the animal experiments, but been found in humans. It is expected that it is possible to overcome and to deviate from ethical controversies over animal experiments through the organoids. In addition, since patient cells are cultured to manufacture organoids, it is attracting attention as a methodology for personalized clinical trials and may be utilized in the future for the development of customized organs.

In the present invention, the "cell culture" means culturing cells isolated from the tissue of a living organism, and depending on the type of cell, the type of medium, temperature conditions, culture medium, etc. follow known methods.

The "derived from the cancer patient" means biological samples obtained from the cancer patient and may include, for example, whole blood, leukocytes, peripheral blood mononuclear cells, buffy coat, blood (including plasma and serum), sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extract and cerebrospinal fluid, but is not limited thereto.

For the purpose of the present invention, the biological sample may be a tissue or cancer cell line isolated from a cancer patient, but is not limited thereto.

The cancer cells refer to cells derived from all types of cancer, and for example, the cancer includes brain tumor, benign astrocytoma, malignant astrocytoma, pituitary adenoma, meningioma, brain lymphoma, brainstem tumor, head and neck tumor, laryngeal cancer, oropharyngeal cancer, nasal cancer, paranasal sinus cancer, nasopharyngeal cancer, salivary gland cancer, hypopharyngeal cancer, thyroid cancer, thoracic tumor, lung cancer, thymic cancer, mediastinal tumor, esophageal cancer, breast cancer, abdominal tumor, stomach cancer, liver cancer, gallbladder cancer, bile duct cancer, pancreatic cancer, small intestine cancer, colon cancer, anal cancer, urinary bladder cancer, kidney cancer, penile cancer, urethral cancer, prostate cancer, cervical cancer, endometrial cancer, ovarian cancer, uterine sarcoma, vaginal cancer, or skin cancer, but is not limited thereto.

The panel may be constituted by various combinations of cultured tumor organoids, and in a specific example, the panel was manufactured in 8 rows × 12 columns in a 96-well strip plate, but is not limited thereto, and may be manufactured by adjusting the number of rows and columns as needed.

Respective columns manufactured above may be easily separated from the plate and may be combined in various shapes to form a plate as needed.

The panel may be prepared of any material that may not be damaged even under liquid nitrogen storage conditions, and prepared of materials such as polypropylene, etc., but is not limited thereto.

The freezing step may be performed by a generally performed freezing method, and may be preferably performed by slow freezing or vitrification, but is not limited thereto.

The tumor organoid may have a diameter of 200 µm or less, preferably 50 µm to 150 µm, but is not limited thereto.

In another aspect of the present invention, the present invention provides a method of screening an anticancer substance using the customized tumor organoid panel.

The method of screening the anticancer substance may include thawing the customized tumor organoid panel; treating the thawed panel with a candidate substance; and measuring anticancer activity.

In the present invention, the term "screening" means specifically selecting only substances having a specific desired property from a candidate group consisting of various substances.

In the present invention, the "candidate substance" refers to an unknown substance that is expected to have inhibitory activity against the growth or metastasis of cancer tissue or to induce the death of cancer-initiating cells. The candidate substance may include a compound, a peptide, a protein, an antibody, and a natural extract, but is not limited thereto.

In the present invention, the candidate substance may be obtained through a synthetic or natural compound library, a biological library, spatially addressable parallel solid phase or solution phase libraries, etc., but is not limited thereto.

The treating of the thawed panel with the test anticancer substance and the measuring of the anticancer activity are a "method for examining the anticancer activity or toxicity" of the test substance, and there is no limitation on its type.

### [Modes]

Hereinafter, the present invention will be described in more detail through Examples. These Examples are to explain the present invention in more detail, and it will be apparent to those skilled in the art that the scope of the present invention is not limited by these Examples in accordance with the gist of the present invention.

### Example 1. Preparation of human-derived tumor organoids

### 1-1. Human liver cancer organoid growth medium

Human liver cancer organoids were cultured in advanced DMEM/F-12 containing 10 mM HEPES (Gibco), 1X GlutaMAX^{™} supplement (Gibco), 1X B-27^{™} supplement (Gibco), 1X N-2 supplement (Gibco), 1% Penicillin-Streptomycin by adding Gastrin 1 (Sigma), N-Acetyl-L-cysteine (Sigma), Nicotinamide (Sigma), A83-01 (Sigma), recombinant human fibroblast growth factor (FGF)-10 (PeproTech), recombinant human hepatocyte growth factor (HGF, PeproTech), human epidermal growth factor recombinant protein (EGF, Gibco), and dexamethasone (Sigma).

### 1-2. Human lung cancer organoid growth medium

Human lung cancer organoids were cultured in DMEM/F-12 containing 1X B-27^{™} supplement (Gibco), 1X N-2 supplement (Gibco), and 1% Penicillin-Streptomycin by adding human EGF recombinant protein (Gibco) and human FGF basic (Gibco).

### 1-3. Human colon cancer organoid growth medium

Human colon cancer organoids were cultured in DMEM/F-12 containing 1X B-27^{™} supplement (Gibco), Primocin (Invivogen), and 1% Penicillin-Streptomycin by adding Gastrin 1 (Sigma), N-Acetyl-L-cysteine (Sigma), Nicotinamide (Sigma), A83-01 (Sigma), and human EGF recombinant protein (Gibco).

### 1-4. Preparation of human liver, lung or colon cancer organoids

Tissues were washed with a medium containing no additives and incubated for 1 hour on a 37°C rotating shaker added with 2X collagenase type II (Gibco) in a 1 : 1 ratio. The tissues were centrifuged at 250 xg for 3 minutes to remove the supernatant, and the cells were suspended in a medium containing no additives. The suspensions were passed through a 100 µm cell strainer, after which the red blood cells were removed. The samples were then centrifuged to remove the supernatant.

The cells were counted and mixed well with a liver cancer, lung cancer, or colon cancer growth medium mixed with Matrigel (Corning) in a 1 : 2 ratio. The cells mixed with the Matrigel were dropped onto the center of the plate and put in a cell incubator to be solidified, and then cultured in a culture incubator (5% CO₂, 37°C) by adding 10 µM Y-27632 Dihydrochloride (Biogems) to the culture medium. The culture medium was replaced every four days with a medium without Y-27632 Dihydrochloride and cultured.

### Example 2. Manufacture of customized tumor organoid panel plate

### 2-1. Manufacture of tumor organoid panel

In addition to the prepared tumor organoids (liver cancer, lung cancer, or colon cancer), various tumor organoids may be configured into a panel to manufacture a customized tumor organoid panel plate.

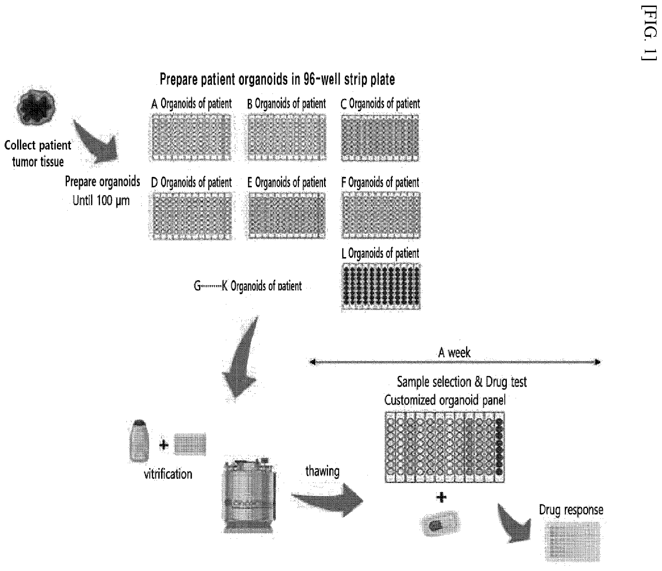

The liver cancer, lung cancer, or colon cancer organoids obtained above were cultured in a 96-well strip plate (8 rows × 12 columns) to have a diameter capable of screening of anticancer substances. The organoids prepared from each patient's tissue were cultured on each plate, and then frozen and stored. Depending on the experimental purpose, tumor organoids were selected, and the required number of columns was taken out from the frozen tumor organoid plate to configure a new tumor organoid panel and the panel was cultured. The cultured tumor organoids were stabilized and then treated with anticancer substances to perform the screening. A drawing showing the panel manufacturing process was attached (see FIG. 1).

### 2-2. Freezing of tumor organoids

The constructed tumor organoid panel plate was frozen using vitrification.

The culturing tumor organoids were obtained by centrifugation. The organoids were washed used phosphate-buffered saline, added with an equilibration solution, and then left at room temperature for 5 minutes. The equilibrium solution was removed by centrifugation, and the organoids were added with a vitrification solution and then stored directly in a liquid nitrogen tank.

### 2-3. Analysis of organoid shape and survival rate according to freezing and thawing

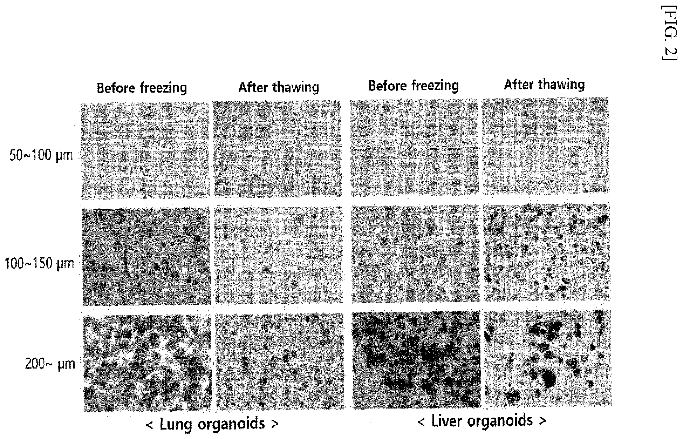

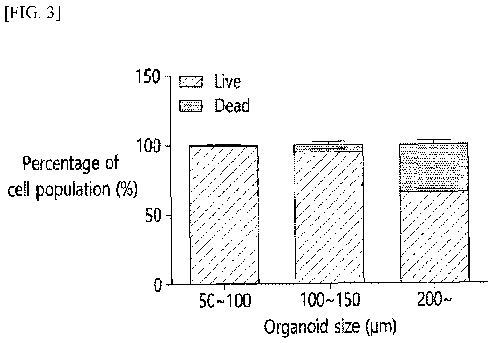

Organoids were cultured to have diameters of 50 to 100 µm, more than 100 to 150 µm, and 200 µm or more and then frozen, respectively. After 3 and 12 months of freezing, the organoids were thawed and then whether the shape thereof was maintained was observed through a microscope and the survival rate was observed, and the results were shown in FIGS. 2 and 3.

As shown in FIG. 2, it was confirmed that when the tumor organoids with a diameter of 200 µm or more were frozen and then thawed, the shape of the organoids was not maintained intact compared to other groups. In addition, as shown in FIG. 3, as a result of confirming the survival rate of the organoids using Calcein-AM and EthD-1, it was confirmed that as the diameter increased, the dead cell ratio increased and organoids of 200 µm or more had damage to the center (data not shown). According to the result, it may be confirmed that when the organoids were frozen to a diameter of less than 200 µm, the survival rate after thawing may be increased.

### Example 3. Anti-cancer drug screening method

### 3-1. Anti-cancer drug screening method

Among the tumor organoid panels manufactured in Example 2 above, the panel may be customized and configured as needed for each patient and cancer type.

As illustrated in FIG. 1, among tumor organoids obtained and prepared from various patients A to L, tumor organoids were selected as needed to form a customized panel.

### 3-2. Evaluation of screening difference according to tumor organoid diameter

### 1) Difference in culture period

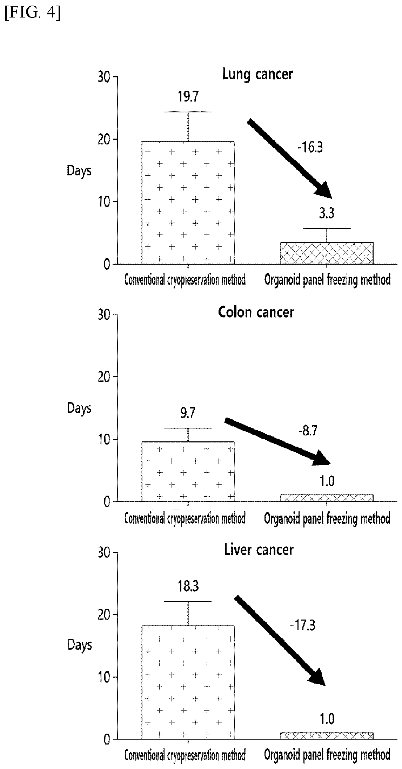

The results of comparing the culture periods before drug treatment in a conventional cryopreservation method and a customized tumor organoid panel freezing method were shown in FIG. 4. As a result, it was confirmed that the customized tumor organoid panel freezing method reached the drug treatment period faster by at least 8.7 days on average and up to 17.3 days than the conventional cryopreservation method in three tumor organoids of lung cancer, colon cancer, and liver cancer.

### 2) Comparison of anticancer efficacy evaluations

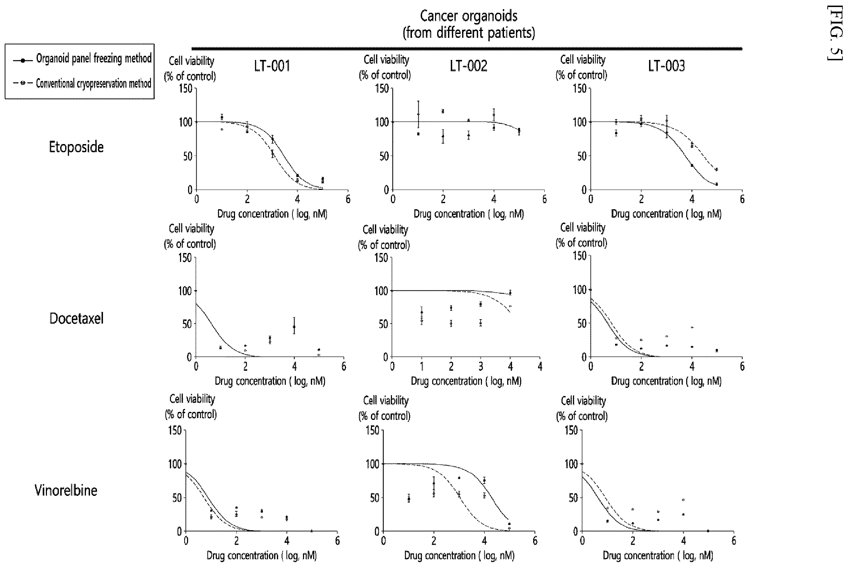

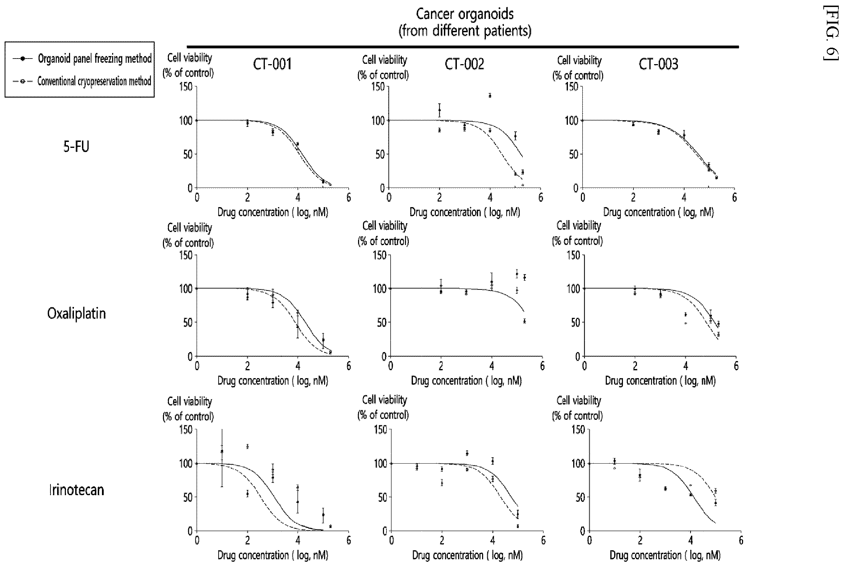

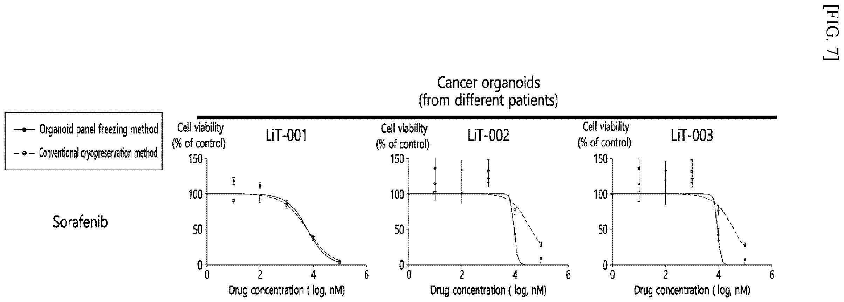

The results of analyzing a correlation were illustrated in FIGS. 5 to 7 by performing anticancer efficacy evaluation according to the conventional cryopreservation method and the customized tumor organoid panel freezing method in three tumor organoids of lung cancer, colon cancer, and liver cancer.

The anticancer efficacy evaluation was conducted with three anticancer drugs of Etoposide, Docetaxel, and Vinorelbine for lung cancer, three anticancer drugs of 5-FU, Oxaliplatin, and Irinotecan for colon cancer, and an anticancer drug of Sorafenib for liver cancer. As a result, as shown in FIGS. 5 to 7, it was confirmed that the anticancer efficacy of the tumor organoid panel manufactured by the freezing method of the present invention could be evaluated similarly to that of tumor organoids prepared by the conventional cryopreservation method.

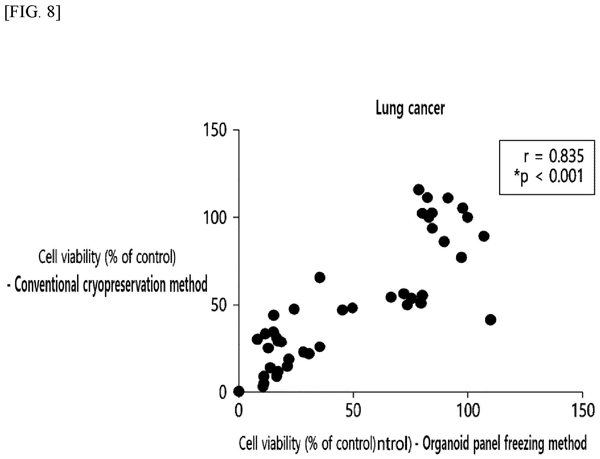

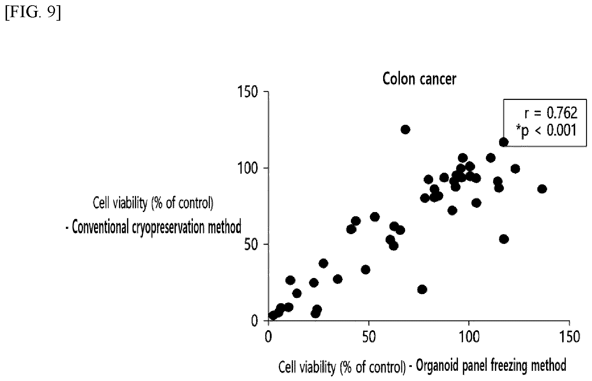

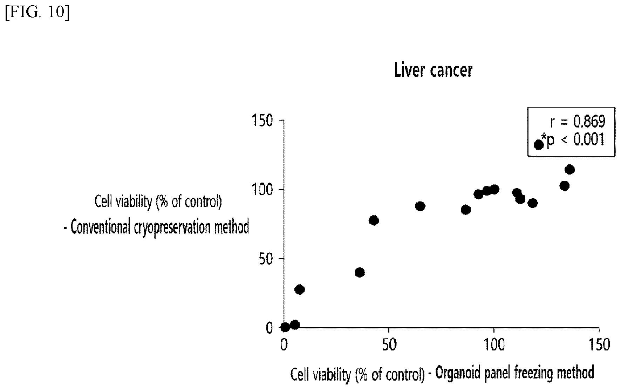

Furthermore, a Spearman's rank correlation test was conducted to statistically confirm the anticancer efficacy. As may be seen from the correlation analysis result for lung cancer (FIG. 8), the correlation analysis result for colon cancer (FIG. 9), and the correlation analysis result for liver cancer (FIG. 10), it was statistically confirmed that r values were 0.835 for lung cancer, 0.762 for colon cancer, and 0.869 for liver cancer, respectively, and the p values were all < 0.001, which had the high correlation with the conventional method, so that there was no problem with efficacy.

As may be seen from the above experimental results, it was confirmed that the tumor organoid panel according to the present invention had a significantly shortened culture period, and not only cultured the organoids in large quantities in a short period of time, but also had no difference in anticancer efficacy experiments compared to organoids prepared by the conventional method, and thus the present invention may be effectively used in the anticancer drug screening method for anticancer treatment.

## Claims

1. A method for manufacturing a customized tumor organoid panel comprising:
(a) culturing tumor organoids derived from a cancer patient;
(b) manufacturing the cultured tumor organoids into a panel;
(c) freezing the manufactured tumor organoid panel; and
(d) combining the frozen tumor organoid panels after thawing.

2. The method for manufacturing the customized tumor organoid panel of claim 1, wherein the cancer of step (a) is selected from the group consisting of brain tumor, benign astrocytoma, malignant astrocytoma, pituitary adenoma, meningioma, brain lymphoma, brainstem tumor, head and neck tumor, laryngeal cancer, oropharyngeal cancer, nasal cancer, paranasal sinus cancer, nasopharyngeal cancer, salivary gland cancer, hypopharyngeal cancer, thyroid cancer, thoracic tumor, lung cancer, thymic cancer, mediastinal tumor, esophageal cancer, breast cancer, abdominal tumor, stomach cancer, liver cancer, gallbladder cancer, bile duct cancer, pancreatic cancer, small intestine cancer, colon cancer, anal cancer, urinary bladder cancer, kidney cancer, penile cancer, urethral cancer, prostate cancer, cervical cancer, endometrial cancer, ovarian cancer, uterine sarcoma, vaginal cancer, and skin cancer.

3. The method for manufacturing the customized tumor organoid panel of claim 1, wherein the freezing of step (c) uses slow freezing or vitrification.

4. The method for manufacturing the customized tumor organoid panel of claim 1, wherein in the freezing of step (c), the tumor organoid has a diameter of 50 µm to 150 µm.

5. A customized tumor organoid panel manufactured by the manufacturing method according to any one of claims 1 to 4.

6. An anticancer drug screening method comprising:
(a) thawing the customized tumor organoid panel of claim 5;
(b) treating the thawed panel with a candidate substance; and
(c) measuring anticancer activity.
